# EUROPEAN PATENT APPLICATION

(11) **EP 4 317 459 A1**
(43) Date of publication of application: **07.02.2024**
(21) Application number: 22188720.1
(22) Date of filing: 04.08.2022
(51) Int. Cl.: C12Q 1/70

(54) **METHOD FOR THE DETECTION OF SARS-COV-2 BY DIGITAL PCR**

(71) Applicant: National and Kapodistrian University of Athens, 10679 Athens (GR)
(72) Inventor: LIANIDOU, Evrykleia, 14575 Dionyssos (GR); STRATI, Areti, 15772 Zografou (GR); PARASKEVIS, Dimitrios, 12462 Chaidari (GR)
(74) Representative: Roukounas, Dimitrios

(57) **Abstract**

A method for the quantification of SARS-CoV-2 in a sample comprising a one-step multiplex reverse transcription digital PCR assay. The method includes the simultaneous amplification of three transcripts of the N gene of SARS-CoV-2, an external control and an internal control.

## Description

### TECHNICAL FIELD OF THE INVENTION

The present invention relates to a method for the detection of SARS-CoV-2 virus in a sample by reverse transcription digital polymerase chain reaction.

### BACKGROUND OF THE INVENTION

Severe acute respiratory syndrome coronavirus 2 (SARS-CoV-2) is a coronavirus which emerged in late 2019 which is the causative pathogen of the coronavirus disease-2019 (COVID-19). The World Health Organisation has reported more than 423 million confirmed cases of SARS-CoV-2 infections and 5.8 million COVID-19 related deaths, by the middle of February 2022. The early characterization of the causative agent of COVID-19 allowed the development of molecular tests for the diagnosis of SARS-CoV-2 infections.

Nucleic acid amplification tests are highly sensitive and specific and are considered as the gold standard methods for SARS-CoV-2 diagnosis. They target one or more genes (i.e. ORF1, N, S genes) and detect current infection.

Digital polymerase chain reaction (dPCR) represents an accurate molecular tool for the absolute quantification of target molecules at very low concentrations. The main principle of dPCR is the partitioning of reaction mixture in an extremely high number of partitions and the detection of fluorescence at the end of PCR amplification. According to Poisson distribution the fraction of positive partitions, determines the number of target copies per unit without the need of calibration curves (Whale, A. S. et al., Fundamentals of Multiplexing with Digital PCR. Biomol. Detect. Quantif. 2016, 10, 15-23). Moreover, amplification inhibitors are divided into different sub-reactions, thus increasing resistance of dPCR reaction to inhibitors and providing high stability (Tan, C. et al., Applications of Digital PCR in COVID-19 Pandemic. View 2021, 2, 20200082). Another important feature of dPCR assays is that sensitivity is not affected by the background of nucleic acid extracts (de Kock, R. et al., Sensitive Detection and Quantification of SARS-CoV-2 by Multiplex Droplet Digital RT-PCR. Eur. J. Clin. Microbiol. Infect. Dis. 2021, 40, 807-813).

Droplet Digital PCR (ddPCR) is a method for performing digital PCR that is based on water-oil emulsion droplet technology. A sample is fractionated into a large number of droplets, typically 20,000 droplets, and PCR amplification is carried out in each individual droplet.

De Kock, R. et al., Sensitive Detection and Quantification of SARS-CoV-2 by Multiplex Droplet Digital RT-PCR. Eur. J. Clin. Microbiol. Infect. Dis. 2021, 40, 807-813 discloses a reverse transcription ddPCR assay for the simultaneous detection of SARS-CoV- 2E, RdRp N viral RNA, and human Rpp30 DNA and GUSB mRNA, for internal nucleic acid (NA) extraction and RT-PCR control. The document discloses a multiplex RT-ddPCR assay for the detection of SARS-COV-2 using two different internal controls but no external control.

Cassinari, K. et al., Assessment of Multiplex Digital Droplet RT-PCR as a Diagnostic Tool for SARS-CoV-2 Detection in Nasopharyngeal Swabs and Saliva Samples. Clin. Chem. 2021, 67, 736-741 discloses a multiplex reverse transcription-digital droplet PCR (RT-ddPCR) assay, targeting 6 SARS-CoV-2 genomic regions. However, no control was used in the assay.

Deiana, M. et al., Assessment of the Direct Quantitation of SARS-CoV-2 by Droplet Digital PCR. Sci. Reports 2020 101 2020, 10, 1-7 discloses the evaluation of 2019-nCoV CDC ddPCR Triplex Probe Assay (BioRad) performance. This commercially available kit includes the detection of two different genomic regions of N gene and Rpp30 as an internal control. The kit does not include an external control.

Telwatte, S. et al., Assays for Measuring the Levels of Subgenomic and Genomic SARS-CoV-2 Transcripts. Methods 2021, S1046-2023(21)00103-1 discloses ddPCR assays targeting seven open reading frames (ORFs) expressed by SARS-CoV-2. This assay does not include the detection of nucleocapsid protein that is the main structural protein of SARS-CoV-2.

### SUMMARY OF THE INVENTION

The present invention provides a highly sensitive and specific in vitro method for the quantification of SARS-CoV-2 in a sample comprising a one-step multiplex reverse transcription digital PCR (RT-dPCR) assay.

The method of the present invention comprises a one-step multiplex RT-dPCR assay for detecting SARS-CoV-2 in a sample and involves the quantification of three different regions of the nucleoprotein (N) gene of SARS-CoV-2, the use of a non-natural synthetic RNA as RNA exogenous control and the use of an endogenous RNA internal control.

The present invention further provides a kit for the quantification of SARS-CoV-2 in a sample.

### BRIEF DESCRIPTION OF THE DRAWINGS

Figure 1 shows the analytical specificity of a one-step five-plex reverse transcription droplet digital PCR according to the invention, depicted in five different 2-D plots corresponding to each gene target (N1, N2, N3, external control, internal control).
Figure 2 shows the linear dynamic range of a one-step five-plex reverse transcription droplet digital PCR according to the invention, in serial 1:2 dilution series from 100 to 25 SARS-CoV-2 RNA copies/reaction for: I) N1 gene transcript, II) N2 gene transcript, III) N3 gene transcript.
Figure 3 shows 2-D plots of samples having varying virus loads and subjected to a one-step five-plex reverse transcription droplet digital PCR according to the invention.
Figure 4 shows the Spearman's correlation between Ct values and absolute number of copies of SARS-COV-2 in identical RNA samples (n=80)

### DETAILED DESCRIPTION OF THE INVENTION

The present invention provides a method for detecting SARS-CoV-2 in a sample which comprises a one-step multiplex reverse transcription digital PCR (RT-dPCR) assay.

Thus, the present invention provides an in vitro method for detecting SARS-CoV-2 in a sample, wherein the method comprises the steps of
a) partitioning the sample into a plurality of partitions, wherein each partition comprises an amplification mixture comprising nucleic acid amplification reagents and
   i) a first forward, a first reverse primer and a first hydrolysis probe which are capable of hybridising to the sequence SEQ ID NO: 1,
   ii) a second forward primer, a second reverse primer and a second hydrolysis probe which are capable of hybridising to the sequence SEQ ID NO: 2,
   iii) a third forward primer, a third reverse primer and a third hydrolysis probe which are capable of hybridising to the sequence SEQ ID NO: 3,
   iv) a fourth forward primer, a fourth reverse primer and a fourth hydrolysis probe which are capable of hybridising to an RNA external control, wherein the external control is a single stranded artificial RNA molecule having from 100 to 150 bases, and
   v) a fifth forward primer, a fifth reverse primer and a fifth hydrolysis probe which are capable of hybridising to an RNA internal control, wherein the internal control is an RNA transcript of a reference gene,
b) performing reverse transcription on the plurality of partitions,
c) performing a digital PCR on the plurality of partitions and
d) providing a conclusion regarding the presence of SARS-CoV-2 in the sample based on whether fluorescence in at least one partition of the plurality of partitions is detected or not.

SEQ ID NO: 1 encodes a first region (N1) of the nucleoprotein (N) gene of SARS-CoV-2. SEQ ID NO: 2 encodes a second region (N2) of the nucleoprotein (N) gene of SARS-CoV-2. SEQ ID NO: 3 encodes a third region (N3) of the nucleoprotein (N) gene of SARS-CoV-2.

According to the present invention, the first forward primer comprises or consists of the sequence 5'-GCGATCAAAACAACGTCGG-3' (SEQ ID NO: 5), the first reverse primer comprises or consists of the sequence 5'- GGTCATCTGGACTGCTATTGGTG -3' (SEQ ID NO: 6), the first hydrolysis probe comprises or consists of the sequence 5'-CCCAAGGTTTACCCAATAATACTGCGTCT-3' (SEQ ID NO: 7); the second forward primer comprises or consists of the sequence 5'-TGATGCTGCTCTTGCTTTGC-3' (SEQ ID NO: 8), the second reverse primer comprises or consists of the sequence 5'-GATTTCTTAGTGACAGTTTGGCCT-3' (SEQ ID NO: 9), the second hydrolysis probe comprises or consists of the sequence 5'-CTGCTTGACAGATTGAACCAGCTTGAGA-3' (SEQ ID NO: 10); the third forward primer comprises or consists of the sequence 5'-TTGGATGACAAAGATCCAAATTTC-3' (SEQ ID NO: 11), the third reverse primer comprises or consists of the sequence 5'-GGCTTGAGTTTCATCAGCCTTC-3' (SEQ ID NO: 12) and the third hydrolysis probe comprises or consists of the sequence 5'-CAAAACATTCCCACCAACAGAGCCTAAAA-3' (SEQ ID NO: 13).

In the method of the present invention all reagents, primers and probes are added to the PCR mixture and then the PCR is carried out, without the need for an additional step, such as in a nested PCR platform. This means that the method of the present invention includes the simultaneous amplification of three transcripts of the N gene of SARS-CoV-2, the external control and the internal control in a one-step PCR. This in turn means that the method includes a one-step multiplex digital PCR assay.

Digital PCR is a PCR technique, well known in the art, which is performed on portions of a sample to determine the presence/absence, concentration, and/or copy number of a nucleic acid target in the sample, based on how many of the sample portions support amplification of the target. In digital PCR, individual nucleic acid molecules are separated from the initial sample into partitions, then amplified to detectable levels. Each partition then provides digital information on the presence or absence of each individual nucleic acid molecule within each partition. When enough partitions are measured using this technique, the digital information can be consolidated to make a statistically relevant measure of starting concentration for the nucleic acid target (analyte) in the sample.

The terms "reverse transcription" refers to a PCR assay utilizing a complementary DNA template produced by reverse transcription of RNA. Reverse transcription PCR allows the analysis of an RNA sample by forming complementary DNA copies of the RNA, such as with a reverse transcriptase enzyme, and PCR amplification using the complementary DNA as a template. The reverse-transcription of RNA may be carried out by techniques well known in the art.

The present invention provides a method which comprises the use of two RNA controls, an internal control and an external control.

The presence of the internal control is important for the evaluation of successful sample collection. The internal control is an RNA transcript of a reference gene. The term "reference gene" refers to a known genome sequence, whether partial or complete, of the organism of a biological sample and which may be used to reference identified sequences from the sample. The reference gene is a gene which is ubiquitous in the cells of the sample. Examples of reference genes include Beta-2-microglobulin (B2M), glyceraldehyde 3-phosphate dehydrogenase (GAPDH), Beta-actin (ACTB), 18S ribosomal RNA (18SrRNA), Ribonuclease P protein subunit p30 (Rpp30), Ribosomal protein L32 (RPL32), Guanine nucleotide binding protein, β-peptide 2-like 1 (GNB2L1), glucuronidase beta (GUSB), ribosomal protein lateral stalk subunit P0 (RPLP0). According to a preferred embodiment, the internal control is an RNA transcript of Beta-2-Microglobulin (B2M) gene, preferably consisting of the sequence SEQ ID NO: 20. Preferably, when the internal control is an RNA transcript of B2M gene consisting of the sequence SEQ ID NO: 20, the fifth forward primer comprises or consists of the sequence 5'-GCCTGCCGTGTGAACCATGT-3' (SEQ ID NO: 17), the fifth reverse primer comprises or consists of the sequence 5'-AAATGCGGCATCTTCAAACCTC-3' (SEQ ID NO: 18) and the fifth hydrolysis probe comprises or consists of the sequence 5'-CATGATGCTGCTTACATGTCTCGATCCCAC-3' (SEQ ID NO: 19).

The presence of the external control is important for quality control assessment of the method of the present invention, since it tests the performance in each discrete reaction of the dPCR assay. The external control is an artificial RNA molecule. The term "artificial RNA" means that the sequence of the RNA molecule does not occur naturally. Preferably, the external control is a single stranded RNA molecule comprising the sequence SEQ ID NO: 4. More preferably, the external control is a single stranded RNA molecule consisting of the sequence SEQ ID NO: 4. Preferably, when the external control comprises or consists of the sequence SEQ ID NO: 4, the fourth forward primer comprises or consists of the sequence 5'-CATAGACCAGTAGTGTTGTCCTTCC-3' (SEQ ID NO: 14), the fourth reverse primer comprises or consists of the sequence 5'-ACTGTAATCGGCGACAACCA-3' (SEQ ID NO: 15) and the fourth hydrolysis probe comprises or consists of the sequence 5'-CCACTACCATCCTCATCCACATATCCCT-3' (SEQ ID NO: 16).

The combination of the internal and the external control in the method of the present invention provides excellent quality control assessment, which is highly important for the standardization and reliability of the results.

According to a preferred embodiment, the dPCR assay is a droplet digital PCR (ddPCR) assay. Droplet digital PCR is a well known method for performing digital PCR which is based on emulsion droplet technology, such as water-oil emulsion technology. The sample is fractionated into a large number of droplets, typically around 20.000, and PCR amplification is carried out in each droplet.

Each hydrolysis probe is labelled with a fluorescent reporter dye at one end and a quencher of fluorescence at the opposite end. Preferably, the fluorescent reporter dye is 6-carboxyfluorescein or (FAM) or hexachlorofluorescein (HEX). In order to identify each target in the multiplex reaction of the present invention, different fluorescent reporter dyes or different mixtures of fluorescent reporter dyes may be used for labelling the hydrolysis probes. Preferably, the quencher is an Iowa Black^{®} quencher or a ZEN^{™} quencher, or a combination of an Iowa Black quencher^{®} and a ZEN^{™} quencher.

According to a preferred embodiment, the first forward primer comprises the sequence 5'-GCGATCAAAACAACGTCGG-3' (SEQ ID NO: 5), the first reverse primer comprises the sequence 5'- GGTCATCTGGACTGCTATTGGTG -3' (SEQ ID NO: 6), the first hydrolysis probe comprises the sequence 5'-CCCAAGGTTTACCCAATAATACTGCGTCT-3' (SEQ ID NO: 7); the second forward primer comprises the sequence 5'-TGATGCTGCTCTTGCTTTGC-3' (SEQ ID NO: 8), the second reverse primer comprises the sequence 5'-GATTTCTTAGTGACAGTTTGGCCT-3' (SEQ ID NO: 9), the second hydrolysis probe comprises the sequence 5'-CTGCTTGACAGATTGAACCAGCTTGAGA-3' (SEQ ID NO: 10); the third forward primer comprises the sequence 5'-TTGGATGACAAAGATCCAAATTTC-3' (SEQ ID NO: 11), the third reverse primer comprises the sequence 5'-GGCTTGAGTTTCATCAGCCTTC-3' (SEQ ID NO: 12), the third hydrolysis probe comprises the sequence 5'-CAAAACATTCCCACCAACAGAGCCTAAAA-3' (SEQ ID NO: 13); the fourth forward primer comprises the sequence 5'-CATAGACCAGTAGTGTTGTCCTTCC-3' (SEQ ID NO: 14), the fourth reverse primer comprises the sequence 5'-ACTGTAATCGGCGACAACCA-3' (SEQ ID NO: 15), the fourth hydrolysis probe comprises the sequence 5'-CCACTACCATCCTCATCCACATATCCCT-3' (SEQ ID NO: 16); the fifth forward primer comprises the sequence 5'-GCCTGCCGTGTGAACCATGT-3' (SEQ ID NO: 17), the fifth reverse primer comprises the sequence 5'-AAATGCGGCATCTTCAAACCTC-3' (SEQ ID NO: 18) and the fifth hydrolysis probe comprises the sequence 5'-CATGATGCTGCTTACATGTCTCGATCCCAC-3' (SEQ ID NO: 19).

According to another preferred embodiment, the first forward primer consists of the sequence 5'-GCGATCAAAACAACGTCGG-3' (SEQ ID NO: 5), the first reverse primer consists of the sequence 5'- GGTCATCTGGACTGCTATTGGTG -3' (SEQ ID NO: 6), the first hydrolysis probe is a mixture of 5'-FAM-CCCAAGGTTTACCCAATAATACTGCGTCT-ZEN / Iowa Black FQ-3' (SEQ ID NO: 7) and 5'-HEX-CCCAAGGTTTACCCAATAATACTGCGTCT-ZEN / Iowa Black FQ-3' (SEQ ID NO: 7); the second forward primer consists of the sequence 5'-TGATGCTGCTCTTGCTTTGC-3' (SEQ ID NO: 8), the second reverse primer consists of the sequence 5'-GATTTCTTAGTGACAGTTTGGCCT-3' (SEQ ID NO: 9), the second hydrolysis probe is 5'-FAM-CTGCTTGACAGATTGAACCAGCTTGAGA - ZEN / Iowa Black FQ'-3' (SEQ ID NO: 10); the third forward primer consists of the sequence 5'-TTGGATGACAAAGATCCAAATTTC-3' (SEQ ID NO: 11), the third reverse primer consists of the sequence 5'-GGCTTGAGTTTCATCAGCCTTC-3' (SEQ ID NO: 12), the third hydrolysis probe is a mixture of 5'- FAM-CAAAACATTCCCACCAACAGAGCCTAAAA - ZEN / Iowa Black FQ -3' (SEQ ID NO: 13) and 5'- HEX-CAAAACATTCCCACCAACAGAGCCTAAAA - ZEN / Iowa Black FQ-3' (SEQ ID NO: 13); the fourth forward primer consists of the sequence 5'-CATAGACCAGTAGTGTTGTCCTTCC-3' (SEQ ID NO: 14), the fourth reverse primer consists of the sequence 5'-ACTGTAATCGGCGACAACCA-3' (SEQ ID NO: 15), the fourth hydrolysis probe is a mixture of 5'- FAM-CCACTACCATCCTCATCCACATATCCCT - ZEN / Iowa Black FQ -3' (SEQ ID NO: 16) and 5'- HEX-CCACTACCATCCTCATCCACATATCCCT - ZEN / Iowa Black FQ-3' (SEQ ID NO: 16); the fifth forward primer consists of the sequence 5'-GCCTGCCGTGTGAACCATGT-3' (SEQ ID NO: 17), the fifth reverse primer consists of the sequence 5'-AAATGCGGCATCTTCAAACCTC-3' (SEQ ID NO: 18) and the fifth hydrolysis probe is 5'- HEX-CATGATGCTGCTTACATGTCTCGATCCCAC - ZEN / Iowa Black FQ -3' (SEQ ID NO: 19).

Preferably, the concentration of the primers in the amplification mixture is from 0.2µM to 2µM, more preferably, from 0.5µM to 1µM and even more preferably, 0.75 µM.

Preferably, the concentration of the probes in the amplification mixture is from 0.05µM to 1.5µM, more preferably, from 0.1µM to 0.6µM and even more preferably, 0.35µM.

Preferably, the amplification in the PCR is carried out at a temperature from 93°C to 95°C, more preferably, at 94°C.

Preferably, the annealing of the primers in the PCR is carried out at a temperature from 60°C to 62°C, more preferably, at 61°C.

The present invention provides a method for the quantification of SARS-CoV-2 which is highly sensitive, specific and very reliable.

The present invention provides also a kit for detecting SARS-CoV-2 in a sample, wherein the kit comprises
a) a first forward primer which comprises or consists of the sequence 5'-GCGATCAAAACAACGTCGG-3' (SEQ ID NO: 5), a first reverse primer which comprises or consists of the sequence 5'- GGTCATCTGGACTGCTATTGGTG -3' (SEQ ID NO: 6), a first hydrolysis probe which comprises or consists of the sequence 5'-CCCAAGGTTTACCCAATAATACTGCGTCT-3' (SEQ ID NO: 7),
b) a second forward primer which comprises or consists of the sequence 5'-TGATGCTGCTCTTGCTTTGC-3' (SEQ ID NO: 8), a second reverse primer which comprises or consists of the sequence 5'-GATTTCTTAGTGACAGTTTGGCCT-3' (SEQ ID NO: 9), a second hydrolysis probe which comprises or consists of the sequence 5'-CTGCTTGACAGATTGAACCAGCTTGAGA-3' (SEQ ID NO: 10),
c) a third forward primer which comprises or consists of the sequence 5'-TTGGATGACAAAGATCCAAATTTC-3' (SEQ ID NO: 11), a third reverse primer which comprises or consists of the sequence 5'-GGCTTGAGTTTCATCAGCCTTC-3' (SEQ ID NO: 12), a third hydrolysis probe which comprises or consists of the sequence 5'-CAAAACATTCCCACCAACAGAGCCTAAAA-3' (SEQ ID NO: 13),
d) a fourth forward primer which comprises or consists of the sequence 5'-CATAGACCAGTAGTGTTGTCCTTCC-3' (SEQ ID NO: 14), a fourth reverse primer which comprises or consists of the sequence 5'-ACTGTAATCGGCGACAACCA-3' (SEQ ID NO: 15), a fourth hydrolysis probe which comprises or consists of the sequence 5'-CCACTACCATCCTCATCCACATATCCCT-3' (SEQ ID NO: 16) and
e) a fifth forward primer which comprises or consists of the sequence 5'-GCCTGCCGTGTGAACCATGT-3' (SEQ ID NO: 17), a fifth reverse primer which comprises or consists of the sequence 5'-AAATGCGGCATCTTCAAACCTC-3' (SEQ ID NO: 18) and a fifth hydrolysis probe which comprises or consists of the sequence 5'-CATGATGCTGCTTACATGTCTCGATCCCAC-3' (SEQ ID NO: 19).

According to another preferred embodiment, the kit comprises
a) a first forward primer which consists of the sequence 5'-GCGATCAAAACAACGTCGG-3' (SEQ ID NO: 5), a first reverse primer which consists of the sequence 5'- GGTCATCTGGACTGCTATTGGTG -3' (SEQ ID NO: 6), a first hydrolysis probe which is a mixture of 5'-FAM-CCCAAGGTTTACCCAATAATACTGCGTCT-ZEN / Iowa Black FQ-3' (SEQ ID NO: 7) and 5'-HEX-CCCAAGGTTTACCCAATAATACTGCGTCT-ZEN / Iowa Black FQ-3' (SEQ ID NO: 7),
b) a second forward primer which consists of the sequence 5'-TGATGCTGCTCTTGCTTTGC-3' (SEQ ID NO: 8), a second reverse primer which consists of the sequence 5'-GATTTCTTAGTGACAGTTTGGCCT-3' (SEQ ID NO: 9), a second hydrolysis probe which is 5'-FAM-CTGCTTGACAGATTGAACCAGCTTGAGA - ZEN / Iowa Black FQ'-3' (SEQ ID NO: 10),
c) a third forward primer which consists of the sequence 5'-TTGGATGACAAAGATCCAAATTTC-3' (SEQ ID NO: 11), a third reverse primer which consists of the sequence 5'-GGCTTGAGTTTCATCAGCCTTC-3' (SEQ ID NO: 12), a third hydrolysis probe which is a mixture of 5'- FAM-CAAAACATTCCCACCAACAGAGCCTAAAA - ZEN / Iowa Black FQ -3' (SEQ ID NO: 13) and 5'- HEX-CAAAACATTCCCACCAACAGAGCCTAAAA - ZEN / Iowa Black FQ-3' (SEQ ID NO: 13),
d) a fourth forward primer which comprises or consists of the sequence 5'-CATAGACCAGTAGTGTTGTCCTTCC-3' (SEQ ID NO: 14), a fourth reverse primer which consists of the sequence 5'-ACTGTAATCGGCGACAACCA-3' (SEQ ID NO: 15), a fourth hydrolysis probe which is a mixture of 5'- FAM-CCACTACCATCCTCATCCACATATCCCT - ZEN / Iowa Black FQ -3' (SEQ ID NO: 16) and 5'- HEX-CCACTACCATCCTCATCCACATATCCCT - ZEN / Iowa Black FQ-3' (SEQ ID NO: 16) and
e) a fifth forward primer which consists of the sequence 5'-GCCTGCCGTGTGAACCATGT-3' (SEQ ID NO: 17), a fifth reverse primer which consists of the sequence 5'-AAATGCGGCATCTTCAAACCTC-3' (SEQ ID NO: 18) and a fifth hydrolysis probe which is 5'- HEX-CATGATGCTGCTTACATGTCTCGATCCCAC - ZEN / Iowa Black FQ -3' (SEQ ID NO: 19).

### EXAMPLES

### Example 1

This example discloses a one-step five-plex reverse transcription droplet digital PCR (RT-ddPCR) assay according to the invention.

### Primers and probes

Target sequence: SEQ ID NO: 1 (N1 region of of the SARS-CoV-2 nucleoprotein (N) gene).
Forward primer: 5'- GCGATCAAAACAACGTCGG -3' ((SEQ ID NO: 5)
Reverse primer: 5'- GGTCATCTGGACTGCTATTGGTG -3' (SEQ ID NO: 6)
Probes: FAM-CCCAAGGTTTACCCAATAATACTGCGTCT-ZEN / Iowa Black FQ (SEQ ID NO: 7), HEX-CCCAAGGTTTACCCAATAATACTGCGTCT-ZEN / Iowa Black FQ (SEQ ID NO: 7)
Target sequence: SEQ ID NO: 2 (N2 region of of the SARS-CoV-2 nucleoprotein (N) gene).
Forward primer: 5'-TGATGCTGCTCTTGCTTTGC-3' (SEQ ID NO: 8)
Reverse primer: 5'-GATTTCTTAGTGACAGTTTGGCCT-3' (SEQ ID NO: 9)
Probe: 5'-FAM-CTGCTTGACAGATTGAACCAGCTTGAGA - ZEN / Iowa Black FQ'-3' (SEQ ID NO: 10)
Target sequence: SEQ ID NO: 3 (N3 region of of the SARS-CoV-2 nucleoprotein (N) gene).
Forward primer: 5'-TTGGATGACAAAGATCCAAATTTC-3' (SEQ ID NO: 11)
Reverse primer: 5'-GGCTTGAGTTTCATCAGCCTTC-3' (SEQ ID NO: 12)
Probes: 5'- FAM-CAAAACATTCCCACCAACAGAGCCTAAAA - ZEN / Iowa Black FQ-3' (SEQ ID NO: 13), 5'- HEX-CAAAACATTCCCACCAACAGAGCCTAAAA - ZEN / Iowa Black FQ-3' (SEQ ID NO: 13)
Target sequence: RNA external control (SEQ ID NO: 4)
Forward primer: 5'-CATAGACCAGTAGTGTTGTCCTTCC-3' (SEQ ID NO: 14)
Reverse primer: 5'-ACTGTAATCGGCGACAACCA-3' (SEQ ID NO: 15)
Probes: 5'- FAM-CCACTACCATCCTCATCCACATATCCCT - ZEN / Iowa Black FQ -3' (SEQ ID NO: 16), 5'- HEX-CCACTACCATCCTCATCCACATATCCCT - ZEN / Iowa Black FQ-3' (SEQ ID NO: 16)
Target sequence: RNA internal control (transcript of B2M gene)
Forward primer: 5'-GCCTGCCGTGTGAACCATGT-3' (SEQ ID NO: 17)
Reverse primer: 5'-AAATGCGGCATCTTCAAACCTC-3' (SEQ ID NO: 18)
Probe: 5'- HEX-CATGATGCTGCTTACATGTCTCGATCCCAC - ZEN / Iowa Black FQ-3' (SEQ ID NO: 19)

### One-step RT-ddPCR

The one-step RT-ddPCR assay was performed using One-step RT-ddPCR Advanced Kit for Probes (Bio-Rad, California, USA). The One-step RT-ddPCR reaction mix consists of the following: 5.0 µL of Supermix, 2.0 µL of reverse transcriptase, 1.0 µL of 300 mM dithiothreitol (DTT), 1.5µL of a primer mix including all the primer pairs (10µM), 2.33µL of a Taqman probe mix (3µM), and H₂O to a final volume of 20µL (the final concentration of primers in the reaction mixture is 0.75µM and the final concentration of the probe mix in the reaction mixture is 0.35 µM). Then, the RT-ddPCR reaction mixture was partitioned into nanoliter-sized droplets using the Bio-Rad QX200^{™} Droplet Generator. Reverse transcription was performed at 50°C for 1 h and for the PCR reaction the following temperature program was applied in the T100^{™} Thermal Cycler: 95°C/10min, 40 cycles of 94°C/30s, and 61°C/60s with a final step at 98°C/10min. A temperature ramp of 2°C/s was fixed on all PCR steps and the lead was heated at 105°C. Following PCR, the 96-well plate was read in the QX200 Droplet Reader (Bio-Rad, California, USA) and the absolute copy number of the five different target transcripts was calculated using the QuantaSoft analysis software (Bio-Rad), according to the Poisson distribution. The one-step five-plex RT-ddPCR assay was evaluated by using as a positive control a commercially available RNA Control that contains full length genomic RNA (N, S, E, and Orf1ab regions) of SARS-CoV-2 (Thermo Scientific^{™} AcroMetrix^{™} Coronavirus 2019 (COVID-19) (RUO, Thermo Fisher Scientific, Rockford, IL, USA).

### Example 2

This example shows the analytical validation of the one-step five-plex RT-ddPCR assay of Example 1.

The analytical performance of the assay was validated by using a mix of RNA transcripts of the SARS-CoV-2 nucleoprotein (N) gene N1 (SEQ ID NO: 1), N2 (SEQ ID NO: 2) and N3 (SEQ ID NO: 3), the RNA external control (RNA-EC) and RNA internal control (RNA-IC) at different copy number concentrations. For this purpose, two different sets of calibrators were prepared. The first set was prepared by mixing in equal copy numbers: the AcroMetrix^{™} Coronavirus 2019 (COVID-19) RNA Control, the RNA-EC and RNA derived from the MCF-7 cell line, where only B2M internal control (IC) is expressed. The calibrators that were generated by serial dilutions, contained 70 copies/µL RNA (CAL1), 25 copies/µL RNA (CAL2) and 12.5 copies/µL RNA (CAL3). The second set was created by mixing in equal copy numbers: RNA derived from a SARS-CoV-2 positive clinical sample, the RNA external control (RNA-EC) and RNA from the MCF-7 cell line. These calibrators were generated by serial dilutions and contained 100 copies/µL RNA (CAL 4), 50 copies/µL RNA (CAL 5) and 25 copies/µL RNA (CAL 6) for each transcript. CAL1, CAL2, CAL3 were used to determine the limit of detection (LOD), limit of quantification (LOQ), intra-assay repeatability, analytical efficiency and precision; CAL4, CAL5, CAL6 were used to determine the inter-assay repeatability and the linear dynamic range of the assay.

### Analytical specificity

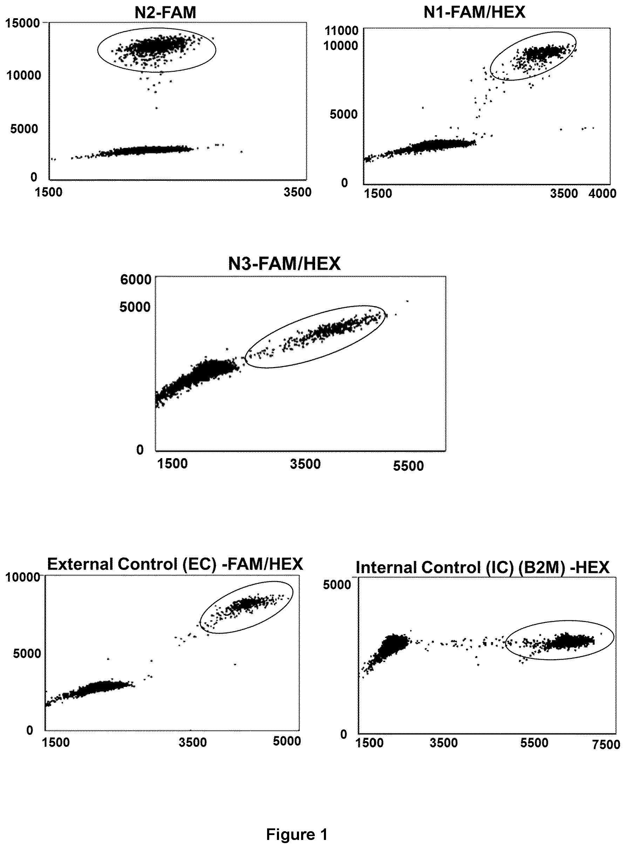

For the evaluation of the analytical specificity of the assay, five DNA synthetic standard solutions, each specific for each target transcript, were prepared to a final concentration of 100 copies/µL. The analytical specificity of the assay was then tested by using as sample only 1 µL of each individual DNA synthetic standard in the absence of all others, representing one specific target transcript in five individual reactions of the five-plex RT-qPCR. Then one-step five-plex RT-ddPCR was performed in the presence of all primers and probes for all transcripts. According to the results, the analytical specificity of the assay is excellent; as can be seen in Figure 1 in a 2-D plot, in these five individual RT-ddPCR reactions only one droplet cluster is obtained corresponding to the specific gene target added in each case.

### Linear dynamic range

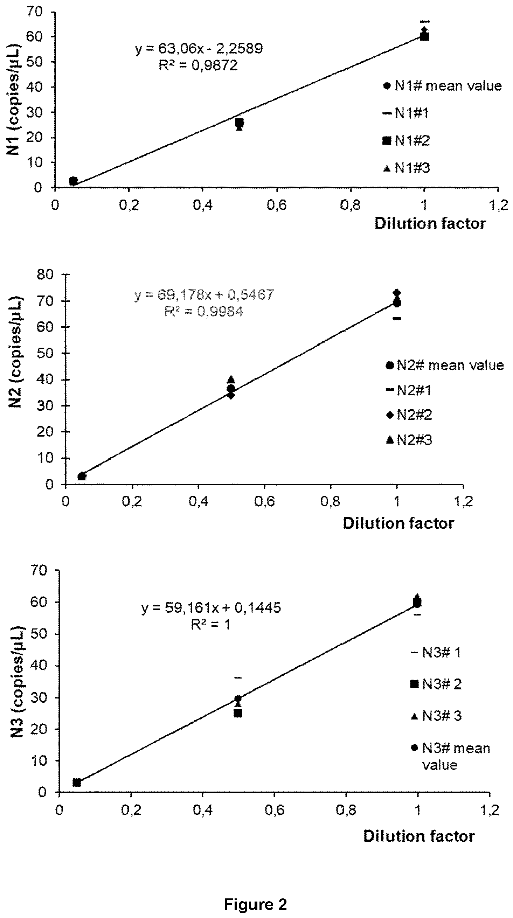

The linear dynamic range (LDR) of each primer/probe set of N gene was evaluated across serial 1:2 dilutions from 100 to 25 SARS-CoV-2 RNA copies/reaction through a linear regression plot representing the absolute number of copies/reaction (Y-axis) in relation to the dilution factor. All reactions were performed in triplicate. The correlation coefficient for all transcripts was higher than 0.98, indicating a precise linear relationship (Figure 2). The linearity of the developed five-plex RT-ddPCR assay was very satisfactory over a broad concentration range for N1, N2, N3 gene transcripts (Figure 2).

### Analytical sensitivity

Limit of Blank (LOB), limit of Detection (LOD) and limit of Quantification (LOQ) of the multiplex RT-ddPCR assay were defined as previously described (Strati, A. et al., Development and Analytical Validation of a Reverse Transcription Droplet Digital PCR (RT-DdPCR) Assay for PD-L1 Transcripts in Circulating Tumor Cells. Clin. Chem. 2021, 67, 642-652). For this purpose, RNA samples derived from 15 healthy donors (HD) were used to determine LOB; all were negative, while RNA-IC was positive. For LOD estimation a commercially available RNA control (AcroMetrix^{™} COVID-19 RNA Control) of known concentration was used at three different concentrations (CAL1:70 copies/reaction, CAL2:25 copies/reaction and CAL3:12.5 copies/reaction) and run in triplicate (Table 1).

The LOD for each of the three distinct genomic areas of the SARS-CoV-2 N gene (N1, N2, N3) was 0.13 copies/µL of ddPCR reaction, (that meaning 2.5 copies/µL of RNA sample input) and its estimation was based on the SD values derived from CAL3 measurements (Table 1). LOQ was set as the lowest positive droplet count that had a CV≤25%. As shown in Table 1, the number of CAL3 transcripts that correspond to the lowest positive droplet count that had a CV ≤25% was 0.5 copies/µL (that meaning 10 copies/µL of RNA sample input).

**Table 1**

| **Intra-assay precision (n=3)** | | | | | | | |
|---|---|---|---|---|---|---|---|
| **Gene target** | **Calibrators** | **Copies/µL (SD)** | **CV %** | **Mean copies per partition (λ*) (SD)** | **CV %** | **Partition number (n) (SD)** | **CV %** |
| **N1** | CAL1^{a} | 3.2 (0.40) | 12 | 2.8 × 10⁻³ (0.31) | 11 | 1.1 × 10⁴ (0.14) | 13 |
| | CAL2^{a} | 1.1 (0.17) | 16 | 9.4 × 10⁻⁴ (1.6) | 17 | 1.1 × 10⁴ (0.087) | 8.1 |
| | CAL3^{a} | 0.49 (0.061) | 13 | 4.1 × 10⁻⁴ (0.54) | 13 | 1.1 × 10⁴ (0.090) | 8.5 |
| **N2** | CAL1^{a} | 3.3 (0.31) | 9.2 | 2.8 × 10⁻³ (0.34) | 12 | 1.1 × 10⁴ (0.14) | 13 |
| | CAL2^{a} | 1.0 (0.15) | 15 | 9.4 × 10⁻⁴ (1.4) | 15 | 1.1 × 10⁴ (0.087) | 8.1 |
| | CAL3^{a} | 0.47 (0.051) | 11 | 4.1 × 10⁻⁴ (0.59) | 14 | 1.1 × 10⁴ (0.090) | 8.5 |
| **N3** | CAL1^{a} | 3.6 (0.35) | 9.6 | 3.4 × 10⁻³ (0.48) | 14 | 1.1 × 10⁴ (0.14) | 13 |
| | CAL2^{a} | 1.0 (0.060) | 5.9 | 8.6 × 10⁻⁴ (0.48) | 5.6 | 1.1 × 10⁴ (0.087) | 8.1 |
| | CAL3^{a} | 0.52 (0.11) | 21 | 3.9 × 10⁻⁴ (0.96) | 25 | 1.1 × 10⁴ (0.090) | 8.5 |
| **RNA-EC** | CAL1^{a} | 3.6 (0.20) | 5.6 | 3.1 × 10⁻³ (0.15) | 5.0 | 1.1 × 10⁴ (0.14) | 13 |
| | CAL2^{a} | 0.63 (0.090) | 14 | 5.4 × 10⁻⁴ (0.79) | 15 | 1.1 × 10⁴ (0.087) | 8.1 |
| | CAL3^{a} | 0.53 (0.11) | 21 | 5.0 × 10⁻⁴ (0.87) | 17 | 1.1 × 10⁴ (0.090) | 8.5 |
| **RNA-IC** | CAL1^{a} | 2.1 (0.17) | 8.2 | 1.8 × 10⁻³ (0.17) | 10 | 1.1 × 10⁴ (0.14) | 13 |
| | CAL2^{a} | 0.41 (0.087) | 21 | 3.5 × 10⁻⁴ (0.74) | 21 | 1.1 × 10⁴ (0.087) | 8.1 |
| | CAL3^{a} | 0.22 (0.023) | 10 | 1.9 × 10⁻⁴ (0.17) | 8.7 | 1.1 × 10⁴ (0.090) | 8.5 |

| **Inter-assay precision (n=4)** | | | | | | | |
|---|---|---|---|---|---|---|---|
| **Gene target** | **Calibrators** | **Copies/µL (SD)** | **CV %** | **Mean copies per partition (λ*) (SD)** | **CV %** | **Partition number (n) (SD)** | **CV %** |
| **N1** | CAL4^{b} | 1.0 × 10¹ | 14 | 8.6 × 10⁻³ (1.2) | 14 | 1.4 × 10⁴ (0.29) | 21 |
| | CAL5^{b} | 5.7 (1.0) | 18 | 4.4 × 10⁻³ (0.89) | 20 | 1.2 × 10⁴ (0.12) | 9.8 |
| | CAL6^{b} | 2.2 (0.33) | 15 | 1.9 × 10⁻³ (0.26) | 14 | 1.2 × 10⁴ (0.12) | 10 |
| **N2** | CAL4^{b} | 1.4 × 10¹ | 7.0 | 1.2 × 10⁻² (0.080) | 6.7 | 1.4 × 10⁴(0.29) | 21 |
| | CAL5^{b} | 6.3 (0.56) | 9.0 | 5.0 × 10⁻³ (0.51) | 10 | 1.2 × 10⁴ (0.12) | 9.8 |
| | CAL6^{b} | 2.4 (0.30) | 13 | 2.1 × 10⁻³ (0.25) | 12 | 1.2 × 10⁴ (0.12) | 10 |
| **N3** | CAL4^{b} | 1.4 × 10¹ | 14 | 1.2 × 10⁻² (0.15) | 13 | 1.4 × 10⁴ (0.29) | 21 |
| | CAL5^{b} | 7.4 (0.43) | 5.8 | 6.2 × 10⁻³ (0.37) | 6.0 | 1.2 × 10⁴ (0.12) | 9.8 |
| | CAL6^{b} | 3.2 (0.59) | 18 | 2.7 × 10⁻³ (0.49) | 19 | 1.2 × 10⁴ (0.12) | 10 |
| **RNA-EC** | CAL4^{b} | 2.8 × 10¹ | 13 | 2.4 × 10⁻² (0.32) | 13 | 1.4 × 10⁴ (0.29) | 21 |
| | CAL5^{b} | 1.4 × 10¹ | 8.1 | 1.2 × 10⁻² (0.12) | 10 | 1.2 × 10⁴ (0.12) | 9.8 |
| | CAL6^{b} | 6.4 (0.50) | 7.9 | 5.4 × 10⁻³ (0.43) | 7.8 | 1.2 × 10⁴ (0.12) | 10 |
| **RNA-IC** | CAL4^{b} | 2.2 × 10¹ | 12 | 1.8 × 10⁻² (0.22) | 12 | 1.4 × 10⁴ (0.29) | 21 |
| | CAL5^{b} | 1.1 × 10¹ | 5.8 | 8.8 × 10⁻³ (1.1) | 13 | 1.2 × 10⁴ (0.12) | 9.8 |
| | CAL6^{b} | 3.5 (0.54) | 15 | 3.0 × 10⁻³ (0.47) | 16 | 1.2 × 10⁴ (0.12) | 10 |

| | | | | | | | |
|---|---|---|---|---|---|---|---|
| a: mix of AcroMetrix^{™} Coronavirus 2019 (COVID-19) RNA Control, EC and RNA derived by MCF-7 cell line, b: mix of RNA derived by a SARS-COV-2(+) clinical sample, the EC and RNA from MCF-7 cell line | | | | | | | |

### Intra and inter-assay repeatability

To evaluate the intra and inter-assay repeatability of the developed assay, the concentration of each gene transcript, the mean copies per partition (λ) and the partition number (n) of each analytical run were calculated, according to the digital MIQE guidelines (Whale, A. et al., The Digital MIQE Guidelines Update: Minimum Information for Publication of Quantitative Digital PCR Experiments for 2020. Clin. Chem. 2020, 66, 1012-1029.). Intra-assay repeatability of the five-plex RT-ddPCR assay was evaluated by analyzing within the same RT-ddPCR run three different concentrations of a commercially available calibrator, with known SARS-CoV2 concentration (CAL1, CAL2, CAL3) in triplicate (Table 1). CV% ranged from 12 to 16% for N1, 9.2 to 15% for N2, 5.9 to 21% for N3, 5.6 to 21% for RNA-EC and 8.2 to 21% for RNA-IC. Reproducibility or inter-assay variance was evaluated by analyzing three samples prepared by mixing in equal copy numbers: RNA derived from a SARS-CoV-2 positive clinical sample, the RNA-EC and RNA-IC (RNA derived from the MCF-7 cell line) (CAL4, CAL5, CAL6) in four separate RT-ddPCR runs performed in four different days (Table 1). CV% in this case ranged from 14 to 18% for N1, 7.0 to 13% for N2, 5.8 to 18% for N3, 7.9 to 13% for RNA-EC and 5.8 to 15% for RNA-IC.

### Analytical recovery

The analytical recovery of the assay was determined using a commercially available RNA control (AcroMetrix^{™} COVID-19 RNA Control) of known concentration at three different concentrations (CAL1:70 copies/reaction, CAL2:25 copies/reaction and CAL3:12.5 copies/reaction). These three samples were quantified by the developed RT-ddPCR assay in three individual reactions in triplicate (Table 2). Analytical recovery (%) was calculated by dividing the copy number of each N gene transcript as found by RT-ddPCR, by the known added copy number in each case and multiplying by 100. For CAL1, analytical recovery ranged from 92.4% to 103%; for CAL2 from 81.6% to 88.0% and for CAL3 from 75.7% to 83.2%, with CV% across reaction replicates ranging from 4.1% to 5.6%, (Table 2).

**Table 2**

| **Calibrators** | **Added Copies/ reaction** | **Found N1 copies±SD (n=3)** | **Found N2 copies±SD (n=3)** | **Found N3 copies±SD (n=3)** | **Found N1, N2, N3, Mean Value±SD (n=9)** | **Recovery (%) (Range, CV%)** |
|---|---|---|---|---|---|---|
| CAL1 | 70 | 65±0.40 | 67±0.31 | 72±0.35 | 68±3.8 | 96.8 (92.4-103, 5.6%) |
| CAL2 | 25 | 22±0.17 | 21±0.15 | 20±0.060 | 21±0.86 | 84.1 (81.6-88.0, 4.1%) |
| CAL3 | 12.5 | 9.7±0.061 | 9.5±0.051 | 10±0.11 | 10±0.48 | 78.9 (75.7-83.2, 4.9%) |

### Quality control

**RNA-EC:** In each reaction, 4,000 copies (200 copies/µL) of RNA-EC were spiked to verify high performance of droplet generation and one-step five-plex RT-ddPCR reaction. 100 copies/µL out of the 200 copies/µL were detected in the FAM channel while the remaining 100 copies/µL were detected in the HEX channel. The mean recovery rate of 100 measurements in each fluorescence amplitude was 97.01±9.0 and the CV% was 9.3%, as shown in Table 3.

**Table 3**

| **Amplitude** | **FAM** | **HEX** | **Total** |
|---|---|---|---|
| **Added (copies/µL)** | 100 | 100 | 200 |
| **Found (copies/µL)** | 97.01 | 97.01 | 194.02 |
| **Recovery %** | 97.01±9.0 | 97.01±9.0 | 97.01±9.0 |
| **SD** | 9.0 | 9.0 | 9.0 |
| **RSD (%)** | 9.3 | 9.3 | 9.3 |

The spiking of RNA-EC in each reaction ensured the good performance of the droplet generator, RT-ddPCR reagents, thermal cycler and QX200 Droplet Reader. The high recovery rate of RNA-EC in all samples tested ensured the reproducibility of all steps of the developed protocol.

**RNA-IC:** Quantification of the RNA-IC (*B2M* transcripts) in each clinical sample verifies the accurate sampling of columnar epithelial cells, and that all experimental steps of the assay worked well. Moreover, the detection of *B2M* transcripts in each sample confirms the absence of false negative results.

The inclusion of external RNA-EC control in all RNA samples in parallel with the simultaneous amplification and detection of *B2M* as an RNA-IC is highly important for the immediate exclusion of potential false negative results, due to low sample quality or pre-analytical errors.

### Example 3

This example shows the quantification of SARS-CoV-2-transcripts in nasopharyngeal and/or oropharyngeal swabs.

The one-step RT-ddPCR of Example 1 was used to detect and quantify SARS-COV-2 transcripts in anonymized and archived (-70°C) remnant RNA extracted from 100 nasopharyngeal swab samples in universal transport medium. Viral RNA was extracted from 300 µL of universal transport medium samples (nasopharyngeal) using the automated CE-IVD TANBead^{®} nucleic acid extraction platform (Taiwan Advanced Nanotech Inc.) according to the manufacturers' instructions The archived samples were selected in order to cover a wide range of viral load as reflected by corresponding Ct-values. The status of the samples (positive or negative) as well as the Ct-values were blinded during the validation experiments.

The one-step five-plex RT-ddPCR of Example 1 was applied by using 5 µL of RNA in each RT-ddPCR reaction. In all samples the same amount of RNA-EC was added. According to the RT-ddPCR results, 19/100 (19.0%) were found negative while 81/100 (81.0%) were found positive for SARS-CoV-2 transcripts. The mean number of copies per µL of RNA of each transcript was 3.5 × 10⁴ for N1 transcript (range: 0.64-9.7 × 10⁵), 3.8 × 10⁴ for N2 transcript (range: 1.28-1.1 × 10⁶) and 3.8 × 10⁴ for N3 transcript (range: 1.92-1.4 × 10⁶).

The concentration of the added RNA-EC found by RT-ddPCR in each sample was almost the same regardless of the viral load of the clinical specimens.

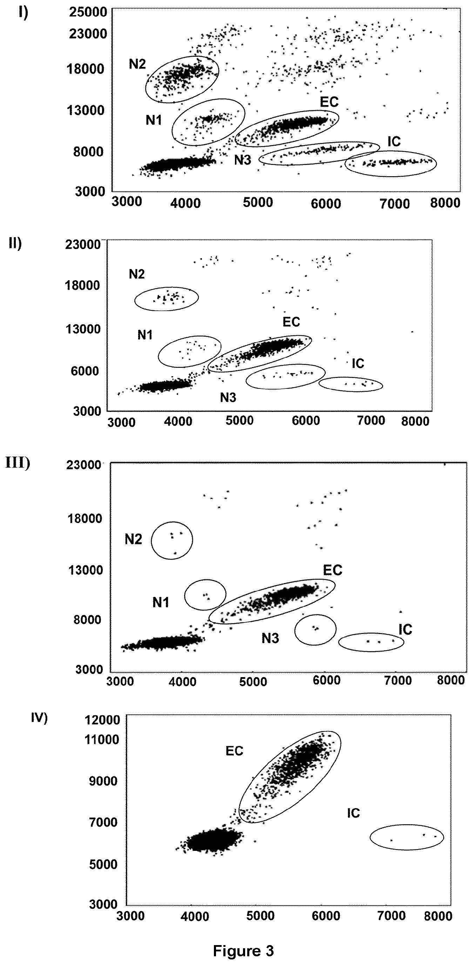

Figure 3 shows four representative 2-D plots are shown: a) a clinical sample with high viral load (I), b) a clinical sample with low viral load (II), c) a clinical sample with ultra-low viral load (III) and d) a negative sample (IV). Copies of RNA-IC were quantified in all clinical samples. The mean number of copies per µL of RNA was 2.1 × 10³(range: 0.76-8.0 × 10⁵).

Similar levels of copies of SARS-COV-2 were found in all three regions, indicating equal efficiencies off all three different single reactions included in the same multiplex setting. Viral load of clinical specimens ranged from very low to very high number of copies, indicating a particular heterogeneity of virus infection. These differences in viral load levels are associated to the duration of the disease and activation of interferon pathway genes.

Quantification of B2M transcripts, used as an RNA-IC in all tested clinical samples revealed that differences in viral load levels are not only due to patient heterogeneity but to differences in sampling recoveries, as columnar epithelial cells express B2M. Biological sampling is a very important parameter in the collection of nasopharyngeal specimens, since suboptimal sampling could contribute to false negative results. In the assay of the present invention, the parallel amplification of B2M in all samples as an RNA-IC and absolute quantification using ddPCR technology provides accurate and sensitive quantification of virus load in clinical specimens, even in cases with limited sample processing.

### Example 4

This example shows a direct comparison of the assay of Example 1 with a commercially available CE-IVD RT-qPCR assay.

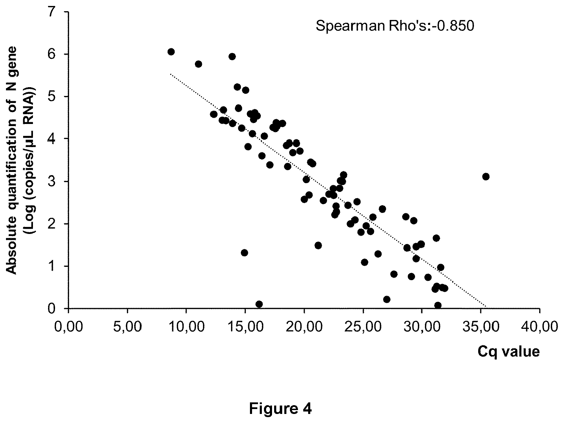

The assay of the Example 1 was directly compared with a commercially available CE-IVD RT-qPCR assay (VIASURE SARS-CoV-2 kit,CerTest BIOTECH Inc). For this purpose, the same 100 RNA samples, collected by nasopharyngeal and/or oropharyngeal swabs were analyzed by both methods. According to the RT-qPCR results, 20/100 (20.0%) were found negative while 80/100 (80.0%) were found positive for SARS-COV-2. The Cq values obtained by RT-qPCR for these 80 positive samples were highly correlated to the absolute number of copies obtained by RT-ddPCR using Spearman's rank correlation coefficient (Figure 4). More specifically, there was a strong negative correlation (Spearman's correlation coefficient = -0.850; p < 0.001) between Cq values and absolute number of copies; lower Cq values were highly correlated to a high number of copies as found by RT-ddPCR while higher Cq values were correlated to a low number of copies. However, there was one sample with low viral load that was found positive using one-step five-plex RT-ddPCR, but negative with RT-qPCR. Therefore, the assay of the present invention represents a significant improvement over the commercially available RT-qPCR assay.

## Claims

1. An in vitro method for detecting SARS-CoV-2 in a sample, wherein the method comprises the steps of
a) partitioning the sample into a plurality of partitions, wherein each partition comprises an amplification mixture comprising nucleic acid amplification reagents and
i) a first forward primer comprising or consisting of the sequence 5'-GCGATCAAAACAACGTCGG-3' (SEQ ID NO: 5), a first reverse primer comprising or consisting of the sequence 5'-GGTCATCTGGACTGCTATTGGTG -3' (SEQ ID NO: 6), a first hydrolysis probe comprising or consisting of the sequence 5'-CCCAAGGTTTACCCAATAATACTGCGTCT-3' (SEQ ID NO: 7),
ii) a second forward primer comprising or consisting of the sequence 5'-TGATGCTGCTCTTGCTTTGC-3' (SEQ ID NO: 8), a second reverse primer comprising or consisting of the sequence 5'-GATTTCTTAGTGACAGTTTGGCCT-3' (SEQ ID NO: 9), a second hydrolysis probe comprising or consisting of the sequence 5'-CTGCTTGACAGATTGAACCAGCTTGAGA-3' (SEQ ID NO: 10),
iii) a third forward primer comprising or consisting of the sequence 5'-TTGGATGACAAAGATCCAAATTTC-3' (SEQ ID NO: 11), a third reverse primer comprising or consisting of the sequence 5'-GGCTTGAGTTTCATCAGCCTTC-3' (SEQ ID NO: 12) a third hydrolysis probe comprising or consisting of the sequence 5'-CAAAACATTCCCACCAACAGAGCCTAAAA-3' (SEQ ID NO: 13),
iv) a fourth forward primer, a fourth reverse primer and a fourth hydrolysis probe which are capable of hybridising to an RNA external control, wherein the external control is a single stranded artificial RNA molecule having from 100 to 150 bases, and
v) a fifth forward primer, a fifth reverse primer and a fifth hydrolysis probe which are capable of hybridising to an RNA internal control, wherein the internal control is an RNA transcript of a reference gene,
b) performing reverse transcription on the plurality of partitions,
c) performing a one-step multiplex digital PCR on the plurality of partitions and
d) providing a conclusion regarding the presence of SARS-CoV-2 in the sample based on whether fluorescence in at least one partition of the plurality of partitions is detected or not.

2. The method according to claim 1, wherein the first forward primer consists of the sequence 5'-GCGATCAAAACAACGTCGG-3' (SEQ ID NO: 5), the first reverse primer consists of the sequence 5'- GGTCATCTGGACTGCTATTGGTG -3' (SEQ ID NO: 6), the first hydrolysis probe consists of the sequence 5'-CCCAAGGTTTACCCAATAATACTGCGTCT-3' (SEQ ID NO: 7); the second forward primer consists of the sequence 5'-TGATGCTGCTCTTGCTTTGC-3' (SEQ ID NO: 8), the second reverse primer consists of the sequence 5'-GATTTCTTAGTGACAGTTTGGCCT-3' (SEQ ID NO: 9), the second hydrolysis probe consists of the sequence 5'-CTGCTTGACAGATTGAACCAGCTTGAGA-3' (SEQ ID NO: 10); the third forward primer consists of the sequence 5'-TTGGATGACAAAGATCCAAATTTC-3' (SEQ ID NO: 11), the third reverse primer consists of the sequence 5'-GGCTTGAGTTTCATCAGCCTTC-3' (SEQ ID NO: 12) and the third hydrolysis probe consists of the sequence 5'-CAAAACATTCCCACCAACAGAGCCTAAAA-3' (SEQ ID NO: 13)

3. The method according to claim 1 or 2, wherein the RNA external control comprises or consists of the sequence SEQ ID NO: 4.

4. The method according to any one of the preceding claims, wherein the fourth forward primer comprises or consists of the sequence 5'-CATAGACCAGTAGTGTTGTCCTTCC-3' (SEQ ID NO: 14), the fourth reverse primer comprises or consists of the sequence 5'-ACTGTAATCGGCGACAACCA-3' (SEQ ID NO: 15) and the fourth hydrolysis probe comprises or consists of the sequence 5'-CCACTACCATCCTCATCCACATATCCCT-3' (SEQ ID NO: 16).

5. The method according to any one of the preceding claims, wherein the fourth forward primer consists of the sequence 5'-CATAGACCAGTAGTGTTGTCCTTCC-3' (SEQ ID NO: 14), the fourth reverse primer consists of the sequence 5'-ACTGTAATCGGCGACAACCA-3' (SEQ ID NO: 15) and the fourth hydrolysis probe consists of the sequence 5'-CCACTACCATCCTCATCCACATATCCCT-3' (SEQ ID NO: 16).

6. The method according to claim 1 or 2, wherein the reference gene is selected from Beta-2-microglobulin (B2M),-glyceraldehyde 3-phosphate dehydrogenase (GAPDH), Beta-actin (ACTB), 18S ribosomal RNA (18SrRNA), Ribonuclease P protein subunit p30 (Rpp30), Ribosomal protein L32 (RPL32), Guanine nucleotide binding protein, β-peptide 2-like 1 (GNB2L1), glucuronidase beta (GUSB), or ribosomal protein lateral stalk subunit P0 (RPLP0).

7. The method according to any one of the preceding claims, wherein the reference gene is B2M.

8. The method according to any one of the preceding claims, wherein the fifth forward primer comprises or consists of the sequence 5'-GCCTGCCGTGTGAACCATGT-3' (SEQ ID NO: 17), the fifth reverse primer comprises or consists of the sequence 5'-AAATGCGGCATCTTCAAACCTC-3' (SEQ ID NO: 18) and the fifth hydrolysis probe comprises or consists of the sequence 5'-CATGATGCTGCTTACATGTCTCGATCCCAC-3' (SEQ ID NO: 19).

9. The method according to any one of the preceding claims, wherein the fifth forward primer consists of the sequence 5'-GCCTGCCGTGTGAACCATGT-3' (SEQ ID NO: 17), the fifth reverse primer consists of the sequence 5'-AAATGCGGCATCTTCAAACCTC-3' (SEQ ID NO: 18) and the fifth hydrolysis probe consists of the sequence 5'-CATGATGCTGCTTACATGTCTCGATCCCAC-3' (SEQ ID NO: 19).

10. The method according to any one of the preceding claims, wherein the digital PCR is droplet digital PCR.

11. The method according to any one of the preceding claims, wherein the concentration of the primers in the amplification mixture is from 0.2µM to 2µM, preferably, from 0.5µM to 1µM and more preferably, 0.75 µM.

12. The method according to any one of the preceding claims, wherein the concentration of the probes in the amplification mixture is from 0.05µM to 1.5µM, preferably, from 0.1µM to 0.6µM and more preferably, 0.35µM

13. The method according to any one of the preceding claims, wherein the amplification in the PCR is carried out at a temperature from 93°C to 95°C, preferably, at 94°C

14. The method according to any one of the preceding claims, wherein the annealing of the primers in the PCR is carried out at a temperature from 60°C to 62°C, preferably, at 61°C.

15. A kit for detecting SARS-CoV-2 in a sample, wherein the kit comprises
a) a first forward primer which comprises or consists of the sequence 5'-GCGATCAAAACAACGTCGG-3' (SEQ ID NO: 5), a first reverse primer which comprises or consists of the sequence 5'- GGTCATCTGGACTGCTATTGGTG - 3' (SEQ ID NO: 6), a first hydrolysis probe which comprises or consists of the sequence 5'-CCCAAGGTTTACCCAATAATACTGCGTCT-3' (SEQ ID NO: 7),
b) a second forward primer which comprises or consists of the sequence 5'-TGATGCTGCTCTTGCTTTGC-3' (SEQ ID NO: 8), a second reverse primer which comprises or consists of the sequence 5'-GATTTCTTAGTGACAGTTTGGCCT-3' (SEQ ID NO: 9), a second hydrolysis probe which comprises or consists of the sequence 5'-CTGCTTGACAGATTGAACCAGCTTGAGA-3' (SEQ ID NO: 10),
c) a third forward primer which comprises or consists of the sequence 5'-TTGGATGACAAAGATCCAAATTTC-3' (SEQ ID NO: 11), a third reverse primer which comprises or consists of the sequence 5'-GGCTTGAGTTTCATCAGCCTTC-3' (SEQ ID NO: 12), a third hydrolysis probe which comprises or consists of the sequence 5'-CAAAACATTCCCACCAACAGAGCCTAAAA-3' (SEQ ID NO: 13),
d) a fourth forward primer which comprises or consists of the sequence 5'-CATAGACCAGTAGTGTTGTCCTTCC-3' (SEQ ID NO: 14), a fourth reverse primer which comprises or consists of the sequence 5'-ACTGTAATCGGCGACAACCA-3' (SEQ ID NO: 15), a fourth hydrolysis probe which comprises or consists of the sequence 5'-CCACTACCATCCTCATCCACATATCCCT-3' (SEQ ID NO: 16) and
e) a fifth forward primer which comprises or consists of the sequence 5'-GCCTGCCGTGTGAACCATGT-3' (SEQ ID NO: 17), a fifth reverse primer which comprises or consists of the sequence 5'-AAATGCGGCATCTTCAAACCTC-3' (SEQ ID NO: 18) and a fifth hydrolysis probe which comprises or consists of the sequence 5'-CATGATGCTGCTTACATGTCTCGATCCCAC-3' (SEQ ID NO: 19).
